# EUROPEAN PATENT SPECIFICATION

(11) **EP 2 034 972 B1**
(45) Date of publication and mention of the grant of the patent: **22.02.2012**
(21) Application number: 07812182.9
(22) Date of filing: 18.06.2007
(51) Int. Cl.: A61K 9/26

(54) **ENTERIC COATED PARTICLES CONTAINING AN ACTIVE INGREDIENT**
BESCHICHTETE DARMPARTIKEL MIT EINEM WIRKSTOFF
PARTICULES À ENROBAGE ENTÉRIQUE CONTENANT UN INGRÉDIENT ACTIF

(30) Priority: 19.06.2006 US 814749 P
(43) Date of publication of application: 18.03.2009
(73) Proprietor: McNeil-PPC, Inc., Skillman, NJ 08558 (US)
(72) Inventor: HUANG, Hugh, Princeton, NJ 08540 (US)
(74) Representative: Kirsch, Susan Edith
(86) International application number: PCT/US2007/071431
(87) International publication number: WO 2007/149801

(56) References cited:
- EP-A- 0 094 117
- WO-A-2004/093801
- US-A- 5 536 507
- US-A- 5 578 316
- US-A1- 2003 152 627

## Description

### BACKGROUND OF THE INVENTION

### 1. Field of the Invention

This invention relates to particles containing an active ingredient and a two-layer enteric coating. The coated particles may be used to make chewable tablets, which can release the active ingredient in the upper intestinal tract or in sustained release fashion.

### 2. Background Information

Pharmaceuticals intended for oral administration are typically provided in solid form as tablets, capsules, pills, lozenges, or granules. Tablets are swallowed whole, chewed in the mouth, or dissolved in the oral cavity. Chewable tablets are typically made from a mixture including active drug particles, and other inactive ingredients (excipients), and are often employed for the administration of pharmaceuticals where it is impractical to provide a tablet for swallowing whole. With chewable tablets, the act of chewing helps to break up the tablet particles as the tablet disintegrates and may increase the rate of absorption by the digestive tract. Chewable tablets are often utilized to improve drug administration in pediatric and geriatric patients.

Certain drug particles are preferably released in the intestines. In order to make tablets having these release characteristics, efforts have focused on coating the cores with a polymeric coating that releases the active ingredient only at a pH above the pH that occurs in the stomach environment, e.g., at pH above about pH 5.5. However, unlike coated compressed tablet cores, chewable dosage forms present unique challenges. First, because chewable dosage forms are chewed as opposed to swallowed, there is the likelihood that the enteric coatings may be separated from the particle cores during mastication. Second, because of the sticky nature of many enteric polymers, much of the coating can adhere to the tablet punch and press surfaces during compression. This problem can be magnified when producing chewable tablets, especially those having embossed letters and numbers. As a result, portions of the chewable tablet and coated particles stick to the embossing sections of the tablet punch, which leads to loss of tablet portions and loss of content uniformity. Disadvantageously, there is often little to no enteric coating left on the particles as such dosage forms pass through the stomach, and the active ingredient thereby is prematurely released into the stomach.

One approach for producing chewable tablets having delayed release of the active may be found in United States Patent No. 4,800,087, which uses an outer microencapsuling polymeric layer containing components such as plasticizers and/or low temperature, soft, plasticizer-like polymers that provide the tablet with the required elasticity. Disadvantageously, the use of these components in the outer layer often causes the tablet to stick to compression tooling, e.g. punches and dies, during production.

US 2003/0152627 A1 discloses tablets comprising coated cores with 5-aminosalicylic acid, the first coating being Eudragit RL, the second coating being Eudragit L30D-55.

It would be desirable to have an oral dosage form that not only may be chewed, but which permits the effective release of the active ingredient in the intestine, as opposed to the stomach, environment.

### Summary of the Invention

The present invention consists, consists essentially of, and/or consists of the particles and chewable dosage forms as claimed herein.

In accordance with this invention, chewable pharmaceutical formulations having an enteric release profile may be made using two, independent layers of polymeric coatings. Beneficially, such dosage forms not only provide sufficient taste-masking properties to the user during mastication, but also permit the effective release the active ingredient in the intestine.

### Detailed Description of the Invention

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified. In addition, all ranges set forth herein are meant to include any combinations of values between the two endpoints, inclusively.

As used herein, the term "substantially covers" or "substantially continuous" means that the coating is generally continuous and generally covers the entire surface of the core or underlying layer, so that little to none of the active ingredient or underlying layer is exposed.

"Tg" as used herein, shall mean glass transition temperature, as determined in accordance with the differential scanning calorimetry procedure set forth in Skoog, Principles of Instrumental Analysis, 716 - 726 (3rd Ed. 1985), and using a 2920 Differential Scanning Calorimeter available from TA Instruments Corporation.

"Enteric" shall mean being able to be dissolved at a pH greater than that of the stomach, i.e., e.g., at a pH of greater than about 5.0 or greater than about 5.5 or greater than about 6.0 or that which is found in the intestines.

"Low temperature film forming materials," as used herein, shall mean a material that forms a film at temperatures less than about 100°C.

"Water soluble" or "water solubilize," as used herein in connection with non-polymeric materials, shall mean from sparingly soluble to very soluble, i.e., not more than 100 parts water required to dissolve 1 part of the non-polymeric, water soluble solute. See Remington, The Science and Practice of Pharmacy, pages 208-209 (2000). "Water soluble" or "water solubilize," as used herein in connection with polymeric materials, shall mean that the polymer swells in water and can be dispersed at the molecular level to form a homogeneous dispersion or colloidal solution. "Water dispersible," as used herein in connection with polymeric materials, shall mean at least a portion of the polymer is removed from the dosage form within 60 minutes after immersion of the dosage form in an aqueous medium such as that used for in-vitro dissolution testing, or gastrointestinal fluids.

The core of the coated particle may comprise any one of a number of active ingredients. Suitable active ingredients broadly include, but are not limited to, pharmaceutically active ingredients, dietary supplements, nutritionals, nutriceuticals, and the like. More specifically these include analgesics, decongestants, expectorants, antitussives, antihistamines, gastrointestinal agents, diuretics, proton-pump inhibitors, bronchodilators, sleep-inducing agents, vitamins, minerals, anti-infectives, nutrients, and mixtures thereof. One class of preferred active ingredients include nonsteroidal anti-inflammatory drugs (NSAIDs), such as ibuprofen, ketoprofen, flurbiprofen, naproxen, diclofenac, rofecoxib, celecoxib, and aspirin. The active ingredient may alternatively be selected from acetaminophen, pseudoephedrine, phenylpropanolamine, chlorpheniramine, dextromethorphan, diphenhydramine, dimenhydrinate, meclizine, famotidine, loperamide, ranitidine, cimetidine, bisacodyl, psyllium, astemizole, loratadine, desloratadine, fexofenadine, cetirizine, antacids, mixtures thereof and pharmaceutically acceptable salts or metabolites thereof. Most preferably, the active ingredient is selected from the group consisting of aspirin, acetaminophen, ibuprofen, pseudoephedrine, dextromethorphan, diphenhydramine, chlorpheniramine, loratadine, calcium carbonate, magnesium hydroxide, magnesium carbonate, magnesium oxide, aluminum hydroxide, mixtures thereof, and pharmaceutically acceptable salts thereof.

Examples of suitable gastrointestinal agents include, but are not limited to, antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotidine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole, antidiarrheals, such as diphenoxylate and loperamide; glycopyrrolate; antiemetics, such as ondansetron, analgesics, such as mesalamine.

In another embodiment of the invention, the active ingredient may be selected from pseudoephedrine, phenylephrine, phenylpropanolamine, chlorpheniramine, dextromethorphan, diphenhydramine, guaifenesin, astemizole, terfenadine, chlophedianol, fexofenadine, loratadine, desloratidine, doxilamine, menthol, norastemizole, cetirizine, benzocaine mixtures thereof and pharmaceutically acceptable salts, esters, isomers, and mixtures thereof.

In another embodiment, the active ingredient may be methylphenidate, modafinil and other active agents suitable for attention deficit hyperactivity disorder or attention deficit disorder, oxybutynin, sidenafil, and pharmaceutically acceptable salts, esters, isomers, and mixtures thereof.

The core of the particle may comprise pure, crystalline active ingredient, or a mixture of active ingredient with optional ingredients, such as binders, surfactants, flavorants, sweeteners, release modifying agents, and other excipients known in the art. Suitable release modifying agents include but are not limited to polymers such as hypromellose, cellulose acetate, ethylcellulose, hydroxypropylcellulose, polyethylene oxides, and polymethacrylates.

The core may be formed using a variety of well known granulation methods, including high sheer wet granulation, spray drying, and fluid bed granulation (including rotary fluid bed granulation). In one embodiment, the particle core is made by fluid bed granulation. The average diameter of the core of the particle may be from about 30 to about 600 microns, or about 50 to about 400 microns.

The first coating layer that is applied to the core is comprised of a film having a Tg less than about 57°C, e.g., less than about 40 °C or about 35°C or between about 30°C to about 40°C. In one embodiment, the first coating layer substantially covers the core surface. Examples of film forming materials suitable for use in the first coating layer include such low temperature, enteric film forming materials including, but not limited to methacrylic acid-methacrylic acid ester copolymers available from Rohm-Haas GmBH, under the tradename, "Eudragit L30D;" cellulose acetate phthalate, such as "Aquateric" which is commercially available from FMC Corporation; polyvinyl acetate phthalate such as "Coateric," which is commercially available from Colorcon, Inc.; shellac; hypromellose phthalate; and derivatives and copolymers thereof; or mixtures thereof.

In one embodiment, the first coating layer may be comprised of an enteric low temperature film forming copolymer comprising a polymethyacrylic acid ester copolymer having a mean molecular weight of about 800,000, and which forms a polymeric film at temperatures less than about 25°C.

The first coating layer is comprised of film forming materials that have a Tg greater than about 70 °C (or "hard polymers") in combination with a plasticizer to produce a film blend having a Tg less than about 40°C. Suitable film forming materials having a Tg greater than about 70 °C include, but are not limited to copolymers of acrylic acid esters, e.g., anionic copolymers of methacrylic acid and methacrylates such as those available from Rohm Pharma under the tradename, "Eudragit." Examples of such hard, polymeric film forming materials include, but are not limited to anionic polymers of methacrylic acid and methacrylates which dissolve at a pH 5.5 and above ("Eudragit L 100-55";) a 30% dispersion of methylmethacrylate-methacrylic acid copolymers having a carboxylic acid group ("Eudragit L 30;") methylmethacrylate-methacrylic acid copolymers having a carboxylic acid group ("Eudragit L100;") and the anionic copolymer of methacrylic acid with carboxylic acid groups ("Eudragit S100"); the "hard polymers" disclosed in United States Patent no. 4,800,087; and derivatives and copolymers thereof, and mixtures thereof.

The first coating layer may optionally contain other, non-enteric film forming materials in an amount, based upon the total weight of the first coating layer, from about 0 percent to about 50 percent, e.g., from about 1 percent to about 40 percent. Examples of such film forming materials suitable for use in the first coating layer include, but not limited to copolymers of methacrylic acid esters, e.g., ethylacrylate methylmethacrylate copolymers available from Rohm Pharma under the tradename, "Eudragit NE 30D" or the cationic polymer of methacrylic acid and methacrylates with carboxylic acid functional groups available from Rohm Pharma under the tradename, "Eudragit E100;" styrene acrylate available from S.C. Johnson under the tradename, "Janocryl 77;" polyethylene oxide available from Union Carbide Corporation under the tradename, "Polyox;" and derivatives and copolymers thereof; and mixtures thereof.

In one embodiment the first coating layer is comprised of, based upon the total weight of the coating materials in the first coating layer, from about 60 percent to about 80 percent of a film forming material having a Tg greater than about 70°C, such as e.g., Eudragit L-100 copolymer, and from about 20 percent to about 45 percent, i.e.. e.g., from about 20 percent to about 40 percent of a plasticizer, such as, e.g., water soluble propylene glycol.

In another embodiment, the first coating layer comprises, based upon the total weight of the first coating layer, at least about 40 percent, i.e., e.g., at least about 50% of an enteric polymer.

Example of suitable plasticizers include, but are not limited to polyethylene glycol; propylene glycol; glycerin; sorbitol; triethyl citrate; tributyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof.

The second coating layer, which is applied to the surface of the first coating layer, is comprised of a film having a Tg greater than about 56°C. In one embodiment, the second coating layer substantially covers the first coating layer. Examples of suitable film forming materials having a Tg greater than about 56°C that are suitable for use in the second coating layer include, but are not limited to the aforementioned copolymers of acrylic acid esters, e.g., anionic polymers of methacrylic acid and methacrylates having carboxy groups such as those available from Rohm Pharma under the tradename, "Eudragit L 100-55"; "Eudragit L 30 D-55;" "Eudragit L100;" and "Eudragit S100;" the "hard polymers* disclosed in United States Patent no. 4,800,087; and derivatives and copolymers thereof, and mixtures thereof. In certain embodiments, these film forming materials are used in combination with a plasticizer to form the second layer that has a Tg greater than about 56 °C.

The second coating layer is comprised of a combination of film forming materials that have a Tg greater than about 70°C and a plasticizer to produce a film blend having a Tg of greater than about 56°C.

In one embodiment, the second coating layer is comprised of a high temperature, film forming copolymer comprising a reaction product of polymethacrylic acid and at least one member of the group consisting of acrylic acid ester and polymethacrylic acid ester, and which forms a polymeric film at temperatures of at least about 30°C.

In one embodiment, the first coating layer possesses an elongation at break value of at least about 70%, and the second coating layer possesses an elongation at break value of between about 1 % and about 50%, when film samples of each layer are independently tested in accordance with that described in the American Society for Testing Materials (ASTM) D882 test measurement. According to this test method, a film sample is cast and cut or stamped using an ASTM D1708 Stamp mold, then inserted into a press such as the Punch Press Model B No. 8463 as produced by the Naef Corporation. The film sample is then placed between two grippers on a texture analyzer, such as the model TA-XT2i *(HR)* available from Texture Technologies Corporation, which elongates the film from two ends and determines the percentage value at break.

The mean particle size of particles that have been coated with both the first coating layer and second coating layer of the present invention may range from about 30 to about 1000 microns, i.e., e.g., from about 50 microns to 600 microns or about 100 microns to 400 microns.

Optional ingredients well known in the art may be added to the composition suitable for use in the first coating layer. Examples of such optional ingredients include, but are not limited to, fillers, including water soluble compressible carbohydrates such as sucrose, mannitol, sorbitol, maltitol, xylitol, erythritol, lactose, and mixtures thereof; non-pH dependent polymers including ethylcellulose, cellulose acetate, hydroxypropyl cellulose, hypromellose, gelatin, gellan gum, xanthan gum, locust bean gum, carageenan, methylcellulose, polyvinyl acetate, polyvinyl alcohol, polyvinyl pyrrolidone, starch, modified starch, maltodextrin, and mixtures thereof, and in particular microcrystalline cellulose, maltodextrin, and starch; sweeteners including aspartame, acesulfame potassium, sucralose and saccharin; disintegrants such as microcrystalline cellulose, starch, sodium starch glycolate, crosslinked polyvinylpyrrolidone, crosslinked carboxymethylcellulose; preservatives, flavors, acidulants, antioxidants, glidants, surfactants, and coloring agents. Any of the optional ingredients set forth above are also suitable for use in second coating layer. Typically, the amount of optional ingredients in the composition suitable for use in either the first coating layer or the second coating layer is, based upon the total wet weight of each respective composition, from about 1 percent to about 40 percent.

Suitable surfactants include both ionic and non-ionic materials from both synthetic and natural origins, including but not limited to lecithin, glyceryl esters, sugar esters, polysorbates, mono and diglycerides of fatty acids, propylene glycol esters, sucrose fatty acid esters, polyoxyethylene derivatives of sorbitan fatty acid esters, and mixtures thereof. Examples of useful polysorbates include sorbitan trioleate, sorbitan monopalmitate, sorbitan monolaurate, propylene glycol monolaurate, glycerol monostearate, diglycerol monostearate, glycerol lactyl-palmitate. Lactic acid derivatives include sodium stearoyl lactylate and calcium stearoyl lactylate. When a surfactant is present in the first coating layer, the level of surfactant is present in an amount, based upon the total weight of the first coating layer, from about 2 percent to about 10 percent.

The first layer coating may be applied to the particle core in the form of a solution using fluidized bed technology, such as Wurster coating or rotor coating. Useful solvents include any of the pharmaceutically suitable organic solvents such as acetone, methanol, ethanol, isopropanol; aqueous solvents such as water; and mixtures thereof. One suitable solvent mixture includes acetone and water at a ratio from about 85:15 to about 95:5.

The thickness of the first layer coating on the core is typically from about 1 micron to about 20 microns, e.g. from about 2 microns to about 15 microns or from about 4 to about 9 microns. The first layer coating may be present in an amount, based upon the total weight of the coated particle before the addition of the second coating thereto, from about 5 percent to about 50 percent, e.g. from about 15 percent to about 25 percent.

The thickness of the second coating layer on the coated core is typically from about 1 to about 20 microns, e.g., from about 2 to about 15 microns or from about 4 microns to about 9 microns. The second coating is present in an amount, based upon the weight of the particle having two coating layers, from about 2 percent to about 40 percent, e.g. from about 3 percent to about 20 percent or about 5 percent to about 15 percent.

The second coating layer may be applied to the coated core via any of the methods set forth above for coating the core with the first coating layer. In one method, the ingredients of the second coating layer are dissolved in a suitable solvent, then the resulting coating solution is applied to the particle core using fluidized bed technology such as Wurster coating or rotor coating. Useful solvents include any of the pharmaceutically suitable organic solvents such as acetone, methanol, ethanol, isopropanol; aqueous solvents such as water; and mixtures thereof. One solvent mixture is ethanol and water. In this embodiment the ratio of ethanol to water in the coating solution is typically from about 10:90 to about 90:10, e.g. from about 50:50 to about 80:20. One skilled in the art may readily appreciate that the coating conditions, such as solution spray rate, drying air temperature and flow rate must be adjusted in order to achieve an equilibrium between the rate of application of the liquid coating solution, and the rate of evaporation of the solvents such that the second coating can be deposited uniformly on the particle to form a complete film without overwetting the particle surface. Details of these methods are well known in the art and set forth in, for example, Lieberman et al., "Pharmaceutical Dosage Forms - Tablets: Volume 3", Chapter 3: Particle Coating Methods (1990).

Tablets comprised of the particles of the present invention may be made by any means known in the art. Conventional methods for tablet production include direct compression ("dry blending"), dry granulation followed by compression, and wet granulation followed by drying and compression. Other methods include the use of compacting roller technology such as a chilsonator or drop roller, or molding, casting, or extrusion technologies. All of these methods are well known in the art, and are described in detail in, for example, Lachman, et al., "The Theory and Practice of Industrial Pharmacy," Chapter 11, (3rd Ed. 1986).

In one embodiment wherein the tablets are formed by the direct compression method, a blend of the particles having two coating layers, and any other appropriate optional ingredients are directly compacted. After blending, a pre-determined volume of particles is filled into a die cavity of a rotary tablet press, which continuously rotates as part of a "die table" from the filling position to a compaction position. The particles are compacted between an upper punch and a lower punch to an ejection position, at which the resulting tablet is pushed from the die cavity by the lower punch and guided to an ejection chute by a stationary "take-off bar.

In embodiments wherein a chewable tablet is desired, the degree of particle compaction is controlled so that the resulting tablets are relatively soft, i.e. they have a hardness of up to about 15 kiloponds per square centimeter (kp/cm²), e.g. from about 1 kp/cm² to about 10 kp/cm² or from about 2 kp/cm² to about 6 kp/cm². "Hardness" is a term used in the art to describe the diametrical breaking strength as measured by conventional pharmaceutical hardness testing equipment, such as a Schleuniger Hardness Tester. In order to compare values across different size tablets, the breaking strength is normalized for the area of the break (which may be approximated as the tablet diameter times the thickness). This normalized value, expressed in kp/cm², is sometimes referred in the art as tablet tensile strength. A general discussion of tablet hardness testing is found in Leiberman et al., 2 Pharmaceutical Dosage Forms - Tablets,. 213 - 217, 327 - 329 (2nd Ed. 1990)(hereinafter "Lieberman").

One of the benefits of the coating composition of the present invention may be demonstrated via content uniformity analysis in accordance with the guidelines outlined in United States Pharmacopoeia (USP) No. 29. In particular, the content uniformity for a particular active ingredient may be determined by measuring the concentration of active ingredient in a random sampling of 10 tablets within a batch in order to determine ff the samples have an overall relative standard deviation (RSD) of less than 6.0%, i.e., less than about 5.0%, or less than about 3.0%, or less than 2.0%, or less than about 1.0%. This would indicate that there would be little to no loss or sticking of tablet material to the tablet punch surfaces during the compression process.

Another method for demonstrating the performance of the coating compositions of the present invention is by determining the amount of active ingredient released in an acidic media at various timepoints, i.e., the lower the amount of active ingredient that is released, the more effective are the enteric properties of the coating composition. Suitable dissolution methods for enteric coatings are disclosed in USP No. 29. Suitable acidic media for use in these methods may include gastric fluid, and 0.1 N hydrochloric acid, as well as acids that include the addition of suitable enzymes indicative of those found in the gastrointestinal tract of animals or man. Various timepoints for analysis may include, but are not limited to, 30 minutes, 60 minutes, 90 minutes, 120 minutes, and 180 minutes, and 240 minutes.

Yet another method for demonstrating the performance of the coating compositions of the present invention is shown by determining the amount of material lost on the tablet press surface upon compressing particles having such coatings, i.e., the lower the amount of material lost, the more effective are the enteric properties of the coating composition. In accordance with this method, the weight of the material that is lost from the intended tablet weight upon compression is measured, and a weight loss of less than about 30 mg, e.g. less than about 20 mg or less than about 10 mg of material is lost upon compression. As a consequence of this potential loss of material, the RSDs when measuring weight variation of tablets using this invention include those less than 5.0%, e.g. less than 3.0%, e.g. less than 2.0%, e.g. less than 1.0%.

The active ingredient is present in the chewable tablet of the present invention in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered.

In another embodiment, a second active ingredient may be present within the matrix of the tablet.

The chewable tablet may also contain other conventional ingredients within the matrix, such as fillers, including water soluble compressible carbohydrates such as dextrose, dextrose monohydrate, sucrose, mannitol, sorbitol, maltitol, xylitol, erythritol, lactose, and mixtures thereof; conventional dry binders including cellulose, cellulosic derivatives, polyvinyl pyrrolidone, starch, modified starch, and mixtures thereof, and in particular microcrystalline cellulose; sweeteners including aspartame, acesulfame potassium, sucralose and saccharin; disintegrants such as microcrystalline cellulose, starch, sodium starch glycolate, crosslinked polyvinylpyrrolidone, crosslinked carboxymethylcellulose; and lubricants, such as magnesium stearate, stearic acid, talc, and waxes. The chewable tablet may also incorporate pharmaceutically acceptable adjuvants, including for example preservatives, flavors, acidulants, antioxidants, glidants, surfactants, and coloring agents.

Particles produced in accordance with the present invention advantageously may be used for immediate release dosage forms which are intended to have an enteric or buffered release profile, e.g., buffered aspirin, because the coating layers do not retard the dissolution of the active ingredient. In one embodiment, the coated particles meet the USP dissolution specifications for the specific active ingredient they contain. In one embodiment of the present invention wherein the active ingredient is aspirin, less than 10% of the active ingredient is released in 120 minutes in 0.1N hydrochloric acid solution and greater than 80% is released in pH 6.8 phosphate buffer at 90 minutes using USP Dissolution Apparatus II (paddle method)

The particles having the two coating layers of the present invention are especially suitable for use in meltaway, and chewable tablet dosage forms that require particles to be both enteric and stress resistant, i.e., the coating will not deform or rupture under compression force during production or upon chewing. A "meltaway dosage form," as used herein, shall mean a dosage form which readily dissolves in the mouth upon ingestion in less than 30 seconds, with little to no chewing. Beneficially, the highly plasticized interior layer provides the particles with sufficient stress-resistant properties. By reducing the amount of plasticizer in the exterior layer, the resulting particles of the present invention possess high tensile strength and non-tackiness on its surface, which therefore prevents sticking problems during tablet compression. In embodiments wherein the chewable tablet is embossed, the surface of the tablet may contain markings of letters and numbers having a depth of at least about 0.2 mm and a width of at least about 1.0 mm.

In one embodiment, the coated active ingredient is blended in a matrix comprising dextrose monohydrate and sucralose. The dextrose monohydrate is present in the tablet in directly compressible form. That is, the dextrose monohydrate has an average particle size of about 100 to about 500 microns, i.e., e.g., from about 100 microns to about 250 microns or from about 150 microns to about 200 microns. Such a particle size is beneficial for imparting adequate flowability and compressibility to the formulation, along with a smooth and creamy mouthfeel according to the invention. The amount of dextrose monohydrate present in the tablet is typically, based upon the total weight of the tablet, from about 15 percent to about 90 percent, i.e., from about 25 percent to about 85 percent of from about 30 percent to about 75 percent.

Specific embodiments of the present invention are illustrated by way of the following examples. This invention is not confined to the specific limitations set forth in these examples, but rather to the scope of the appended claims. Unless otherwise stated, the percentages and ratios given below are by weight.

### Examples

### EXAMPLE 1: Preparation of Enteric Release Coating Solutions

A first coating solution (I) was prepared by dispersing propylene glycol, anionic copolymer of methacrylic acid and methacrylates (Eudragit L 30 D-55), and glycerol monostearate (GMS) in purified water under ambient conditions, so that the finished dispersion contained 25.8% of the coating materials. The weight percentage of coating materials were, based upon the dried weight of final coating, as set forth in Table A below:

**Table A- Composition of Coating Solution (I.)**

| ***Component Name*** | ***Amount Present (wt. % based on dried coating)*** |
|---|---|
| Eudragit L 30 D-55* (30% Solid dispersion) | 68.2 % |
| Propylene Glycol | 27.3% |
| GMS | 4.5% |

| | |
|---|---|
| *available from Rohm America, LLC. | |

In a separate container a second coating solution (II) was prepared by dispersing propylene glycol, anionic copolymer of methacrylic acid and methacrylates (Eudragit L 30 D-55), and glycerol monostearate (GMS) in purified water under ambient conditions, so that the finished dispersion contained 20.6% of the coating materials. The weight percentage of coating materials were, based upon the dried weight of final coating, as set forth in Table B below:

**Table B- Composition of Coating Solution (II.)**

| ***Component Name*** | ***Amount Present (wt.* % *based* on *dried coating)*** |
|---|---|
| Eudragit L 30 D-55* (30% Solid dispersion) | 86.2% |
| Propylene Glycol | 10.3% |
| GMS | 3.4% |

### EXAMPLE 2: Preparation of Coated Active Ingredient Granules

Preparation of Coated Aspirin Granules: 2000 g of aspirin crystals (Rhodine 3025, 20 mesh crystals) were sequentially and independently coated with the first coating solution I and then the second coating solution II described in Example 1 at a spray rate of about 20 g/min in a Glatt GPCG-5/9 fluid bed unit with a Wurster insert under product temperature conditions of about 30°C to about 32°C and an atomization air pressure of 2.6 bar.

The resulting coated aspirin granules contained, based upon the total dry weight of the granules and the two enteric coating layers, about 41% of both enteric coating layers and about 8.5% of just the second enteric coating layer. Prior to applying the second coating layer, the resulting coated aspirin granules contained, based upon the total dry weight of the granules coating with the first coating layer, about 35.5 % of the first coating layer.

### EXAMPLE 3: Production of Tablets for Evaluation Thereof

All materials set forth in Table C below (except the encapsulated aspirin) were manually passed through a 30 mesh screen. The resulting blend and the encapsulated aspirin were placed into a 4 quart V-Blender and mixed for 5 minutes.

**Table C: Components of Chewable Particles**

| **Ingredients** | **Percent** | **mg/tab** |
|---|---|---|
| | **(w/w)** | |
| Encapsulated Aspirin (60% active) | 17.83 | 137.26 |
| Saccharin | 1.00 | 7.7 |
| Crospovidone | 0.52 | 4.0 |
| Orange Flavor | 1.10 | 8.45 |
| Stearic Acid | 0.80 | 6.16 |
| Dextrose Monohydrate | 76.61 | 589.88 |
| Citric Acid | 0.50 | 3.85 |
| Pregelatinized Starch | 1.65 | 12.7 |
| **TOTAL** | 100.0 | 770.00 |

The resulting blend was then removed from the blender and compressed on a rotary tablet press at 60 rpm using 1/2" round diameter flat faced beveled edge tablet tooling in order to yield tablets having a weight of 770 mg and a hardness range of about 4 to about 7 kiloponds as determined by the Hardness test set forth in Lieberman, and a thickness of about 5.4 to about 5.9 millimeters. At least 1500 tablets were prepared in accordance with this method, and no visible sticking of material to the tablet punch surfaces was observed.

### EXAMPLE 4: Analysis of Dissolution Data

Aspirin Dissolution Test: An aspirin tablet produced in Example 3 was placed into a USP Type I apparatus (baskets, 100 RPM) containing 1000 ml of a 0.1 N hydrochloric acid solution (acid stage) at 37°C for 120 minutes. After removing the aspirin tablet, 20 mL of the resulting solution was analyzed for aspirin concentration ("Acid Sample")

The same aspirin was then placed into an independent USP Type I apparatus (baskets, 100 RPM) containing 1000 ml of a pH 6.8 phosphate buffer (buffer stage) at 37°C for an additional 90 minutes. After removing the aspiring, 20 mL of the resulting solution was ten analyzed for aspirin concentration ("Basic Sample").

Both the Acid Sample and the Basic Sample were analyzed for aspirin quantity in comparison with a standard solution prepared at the theoretical concentration to achieve a 100% active release for the acid stage timepoint and 100% active release for the buffer stage timepoint, respectively. The samples were analyzed using a 3 x 150 mm C18 µ-Bondapak Waters^{®} high pressure liquid chromatograph (HPLC) equipped with a UV detector set at a wavelength of 277 nm, an injection volume of 10µL, a flow rate of 1.0 mL/minute, and a mobile phase having a 65:35 ratio of 0.1M Ammonium Phosphate Monobasic (pH of 2.90): Acetonitrile. The approximate retention volume for the aspirin peak was 3.0mL.

This procedure was independently repeated for 6 tablets produced in accordance with the procedure of Example 3.

The results showed that the tablets had less than 10% aspirin release in the acid environment and 100% aspirin release in the pH 6.8 buffer environment. This Example therefore showed that the coatings of the present invention would provide effective enteric release properties to the aspirin particles.

### EXAMPLE 5: Preparation and Analysis of Tg in Polymer Films

Five films having the formulas set forth in Table D below were independently prepared by combining about 0.5 mL of deionized water with propylene glycol (available from Dow Chemical Corporation) in a beaker at room temperature. Eudragit L30D-55 polymer available from Degussa Corporation was added thereto with mixing. The five mixtures were then placed in a 40°C oven and allowed to dry for at least 12 hours. The dried films were analyzed for Tg using the 2920 Differential Scanning Calorimeter available from TA Instruments Corporation. The results are set forth in Table D below:

**Table D**

| Film | Weight of Eudragit L30D-55 (mg) | Weight of Propylene Glycol (mg) | Ratio of Eudragit L30D: Propylene Glycol | Tg (°C) |
|---|---|---|---|---|
| 1 | 1895 | 30 | 95:5 | 82.5 |
| 2 | 900 | 30 | 90:10 | 56.5 |
| 3 | 399 | 30 | 80:20 | 29.9 |
| 4 | 233 | 30 | 70:30 | 1.3 |
| 5 | 200 | 40 | 60:40 | -2.7 |

## Claims

1. An enterically coated particle comprising
a) a particle core containing an active ingredient;
b) a first layer that substantially covers the core and has a surface; and
c) a second layer on the surface of the first layer,
wherein the first layer is comprised of, based upon the total dry weight of the first layer:
a) from 60 percent to 80 percent of methacrylic acid-methyl methacrylate copolymer having a Tg greater than about 70°C; and
b) hom 20 percent to 40 percent of a plasticizer; and
wherein the second layer is comprised of, based upon the total dry weight of the second layer:
a) from 80 percent to 95 percent of a film forming material having a Tg greater than about 70 °C; and
b) hom 5 percent to 20 percent of a plasticizer;
wherein the first layer has a Tg less than about 40 °C and the second layer has a Tg greater than about 56°C.

2. The particle of claim 1, wherein the second layer substantially covers the first layer.

3. The particle of claim 1 or 2, wherein the first layer is present in an amount, based upon the total weight of the coated particle, from 5% to 50% and the second layer is present in an amount, based upon the total weight of the coated particle, from 3% to 20%.

4. The particle of claim 1 -3, wherein the active ingredient is selected from the group consisting of a nonsteroidal anti-inflammatory drug, aspirin, acetaminophen, pseudoephedrine, phenylpropanolamine, chlorpheniramine, dextromethorphan, diphenhydramine, dimenhydrinate, meclizine, famotidine, loperamide, ranitidine, cimetidine, astemizole, loratadine, desloratadine, fexofenadine, cetirizine, antacids, modafinil, methylphenidate, oxybutynin, pharmaceutically acceptable salts thereof, metabolites thereof, and mixtures thereof.

5. The particle of claim 1-4, wherein the first layer is comprised of methacrylic acid-methacrylic acid ester copolymers; cellulose acetate phthalate; polyvinyl acetate phthalate; shellac; hypromellose phthalate; or derivatives, copolymers or mixtures thereof.

6. The particle of claim 1-5, or the chewable tablet of claim 5, wherein the first layer is comprised of a methacrylic acid-methylacrylic acid ester copolymer having a mean molecular weight of about 800,000.

7. The particle of any preceeding claim, wherein the plasticizer is selected from the group consisting of polyethylene glycol; propylene glycol; glycerin; triethyl citrate; tributyl citrate; dibutyl sebecate; vegetable oils; surfactants; mono acetate of glycerol; diacetate of glycerol; triacetate of glycerol; triacetin; acetyltributyl citrate; dibutylsuccinate; hydrogenated castor oil; fatty acids; substituted triglycerides; and glycerides; and mixtures thereof.

8. The particle of any preceding claim, wherein the first layer possesses an elongation at break value of at least about 70%.

9. The particle of any preceding claim, wherein the second layer is comprised of copolymers of acrylic acid esters.

10. The particle of any preceding claim, wherein the second layer is comprised of a film forming copolymer comprising a reaction product of polymethacrylic acid and at least one member of the group consisting of acrylic acid ester and polymethacrylic acid ester.

11. The particle of any preceding claim, wherein the first layer comprises, based upon the total weight of the first layer, at least about 50 percent of an enteric polymer.

12. A tablet comprised of the particles of any preceding claim.

13. A chewable tablet comprised of the particles of any preceding claim.

14. The chewable tablet of claim 13, wherein the plasticizer is selected from the group consisting of polyethylene glycol; propylene glycol; glycerin; sorbitol; triethyl citrate; tributyl citrate; dibutyl sebecate; vegetable oils; surfactants; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and mixtures thereof.

15. The chewable tablet of claim 13, wherein the first layer comprises, based upon the total weight of the first layer, at least about 20 percent of an enteric polymer.

16. A meltaway dosage form comprised of the particles of one of claims 1-11.

17. A chewable tablet comprised of at least one first particle of any one of claims 1-11 and at least one second particle;
said second particle comprising a second particle core containing a second active ingredient.

## Patentansprüche

1. Magensaftresistent überzogenes Teilchen, umfassend
a) einen Teilchenkern, der einen Wirkstoff enthält;
b) eine erste Schicht, die den Kern im Wesentlichen bedeckt und eine Oberfläche aufweist;
c) eine zweite Schicht auf der Oberfläche der ersten Schicht,
wobei die erste Schicht, bezogen auf das Gesamt-Trockengewicht der ersten Schicht,
a) 60 Prozent bis 80 Prozent Methacrylsäure-Methylmethacrylat-Copolymer mit einer Tg von mehr als etwa 70 °C; und
b) 20 Prozent bis 40 Prozent eines Weichmachers
umfasst; und
wobei die zweite Schicht, bezogen auf das Gesamt-Trockengewicht der zweiten Schicht,
a) 80 Prozent bis 95 Prozent eines filmbildenden Materials mit einer Tg von mehr als etwa 70 °C; und
b) 5 Prozent bis 20 Prozent eines Weichmachers;
umfasst,
wobei die erste Schicht eine Tg von weniger als etwa 40 °C und die zweite Schicht eine Tg von mehr als etwa 56 °C aufweist.

2. Teilchen gemäß Anspruch 1, wobei die zweite Schicht die erste Schicht im Wesentlichen bedeckt.

3. Teilchen gemäß Anspruch 1 oder 2, wobei die erste Schicht in einer Menge von 5 % bis 50 %, bezogen auf das Gesamtgewicht des überzogenen Teilchens, vorhanden ist und die zweite Schicht in einer Menge von 3 % bis 20 %, bezogen auf das Gesamtgewicht des überzogenen Teilchens, vorhanden ist.

4. Teilchen gemäß Anspruch 1-3, wobei der Wirkstoff ausgewählt ist aus der Gruppe bestehend aus einem nichtsteroiden entzündungshemmenden Arzneimittel, Aspirin, Acetaminophen, Pseudoephedrin, Phenylpropanolamin, Chlorpheniramin, Dextromethorphan, Diphenhydramin, Dimenhydrinat, Meclizin, Famotidin, Loperamid, Ranitidin, Cimetidin, Astemizol, Loratadin, Desloratadin, Fexofenadin, Cetirizin, Antazida, Modafinil, Methylphenidat, Oxybutynin, pharmazeutisch verträglichen Salzen davon, Metaboliten davon und Gemischen davon.

5. Teilchen gemäß Anspruch 1-4, wobei die erste Schicht Methacrylsäure-Methacrylsäureester-Copolymere; Celluloseacetatphthalat; Polyvinylacetatphthalat; Schellack; Hypromellosephthalat; oder Derivate, Copolymere oder Gemische davon umfasst.

6. Teilchen gemäß Anspruch 1-5 oder kaubare Tablette gemäß Anspruch 5, wobei die erste Schicht ein Methacrylsäure-Methylacrylsäureester-Copolymer mit einem mittleren Molekulargewicht von etwa 800.000 umfasst.

7. Teilchen gemäß einem der vorstehenden Ansprüche, wobei der Weichmacher ausgewählt ist aus der Gruppe bestehend aus Polyethylenglycol; Propylenglycol; Glycerin; Triethylcitrat; Tributylcitrat; Dibutylsebecat; Pflanzenölen; grenzflächenaktiven Mitteln; Monoacetat von Glycerol; Diacetat von Glycerol; Triacetat von Glycerol; Triacetin; Acetyltributylcitrat; Dibutylsuccinat; hydriertem Castoröl; Fettsäuren; substituierten Triglyceriden; und Glyceriden; und Gemischen davon.

8. Teilchen gemäß einem der vorstehenden Ansprüche, wobei die erste Schicht einen Wert der Bruchdehnung von wenigstens etwa 70 % aufweist.

9. Teilchen gemäß einem der vorstehenden Ansprüche, wobei die zweite Schicht Copolymere von Acrylsäureestern umfasst.

10. Teilchen gemäß einem der vorstehenden Ansprüche, wobei die zweite Schicht ein filmbildendes Copolymer, umfassend ein Reaktionsprodukt von Polymethacrylsäure und wenigstens einem Mitglied der Gruppe bestehend aus Acrylsäureester und Polymethacrylsäureester, umfasst.

11. Teilchen gemäß einem der vorstehenden Ansprüche, wobei die erste Schicht, bezogen auf das Gesamtgewicht der ersten Schicht, wenigstens etwa 50 Prozent eines magensaftresistenten Polymers umfasst.

12. Tablette, umfassend die Teilchen gemäß einem der vorstehenden Ansprüche.

13. Kaubare Tablette, umfassend die Teilchen gemäß einem der vorstehenden Ansprüche.

14. Kaubare Tablette gemäß Anspruch 13, wobei der Weichmacher ausgewählt ist aus der Gruppe bestehend aus Polyethylenglycol; Propylenglycol; Glycerin; Triethylcitrat; Tributylcitrat; Dibutylsebecat; Pflanzenölen; grenzflächenaktiven Mitteln; Monoacetat von Glycerol; Diacetat von Glycerol; Triacetat von Glycerol; natürlichen Gummis; Triacetin; Acetyltributylcitrat; Diethyloxalat; Diethylmaleat; Diethylfumarat; Diethylmalonat; Dioctylphthalat; Dibutylsuccinat; Glycerolbutyrat; hydriertem Castoröl; Fettsäuren; substituierten Triglyceriden und Glyceriden; und Gemischen davon.

15. Kaubare Tablette gemäß Anspruch 13, wobei die erste Schicht, bezogen auf das Gesamtgewicht der ersten Schicht, wenigstens etwa 20 Prozent eines magensaftresistenten Polymers umfasst.

16. Wegschmelzende Dosierungsform, umfassend die Teilchen gemäß einem der Ansprüche 1-11.

17. Kaubare Tablette, umfassend wenigstens ein erstes Teilchen gemäß einem der Ansprüche 1-11 und wenigstens ein zweites Teilchen;
wobei das zweite Teilchen einen zweiten Teilchenkern umfasst, der einen zweiten Wirkstoff enthält.

## Revendications

1. Particule à enrobage gastrorésistant, comprenant :
a) un coeur de particule contenant un ingrédient actif ;
b) une première couche qui recouvre sensiblement le coeur et possède une surface ; et
c) une deuxième couche à la surface de la première couche,
où la première couche est constituée, sur la base du poids sec total de la première couche :
a) de 60% à 80% d'un copolymère d'acide méthacrylique-méthacrylate de méthyle ayant une Tg supérieure à environ 70°C ; et
b) de 20% à 40% d'un plastifiant ; et
où la deuxième couche est constituée, sur la base du poids sec total de la deuxième couche :
a) de 80% à 95% d'un matériau filmogène ayant une Tg supérieure à environ 70°C ; et
b) de 5% à 20% d'un plastifiant ;
où la première couche possède une Tg inférieure à environ 40°C et la deuxième couche possède une Tg supérieure à environ 56°C.

2. Particule selon la revendication 1, dans laquelle la deuxième couche recouvre sensiblement la première couche.

3. Particule selon la revendication 1 ou 2, dans laquelle la première couche est présente selon une quantité, sur la base du poids total de la particule enrobée, allant de 5% à 50% et la deuxième couche est présente selon une quantité, sur la base du poids total de la particule enrobée, allant de 3% à 20%.

4. Particule selon les revendications 1-3, dans laquelle l'ingrédient actif est choisi dans le groupe constitué par les anti-inflammatoires non stéroïdiens, l'aspirine, l'acétaminophène, la pseudoéphédrine, la phénylpropanolamine, la chlorphéniramine, le dextrométhorphane, la diphénhydramine, le diménhydrinate, la méclizine, la famotidine, le lopéramide, la ranitidine, la cimétidine, l'astémizole, la loratadine, la desloratadine, la fexofénadine, la cétirizine, les antiacides, le modafinil, le méthylphénidate, l'oxybutynine, les sels pharmaceutiquement acceptables de ceux-ci, les métabolites de ceux-ci, et des mélanges de ceux-ci.

5. Particule selon les revendications 1-4, dans laquelle la première couche est constituée de copolymères d'acide méthacrylique-ester d'acide méthacrylique ; d'acétate-phtalate de cellulose ; d'acétate-phtalate de polyvinyle ; de gomme laque ; de phtalate d'hypromellose ; ou de dérivés, copolymères ou mélanges de ceux-ci.

6. Particule selon les revendications 1-5, ou comprimé croquable selon la revendication 5, dans laquelle la première couche est constituée d'un copolymère d'acide méthacrylique-ester d'acide méthacrylique ayant un poids moléculaire moyen d'environ 800 000.

7. Particule selon l'une quelconque des revendications précédentes, dans laquelle le plastifiant est choisi dans le groupe constitué par le polyéthylène glycol ; le propylène glycol ; le glycérol ; le citrate de triéthyle ; le citrate de tributyle ; le sébacate de dibutyle ; les huiles végétales ; les agents tensioactifs ; le mono-acétate de glycérol ; le diacétate de glycérol ; le triacétate de glycérol ; la triacétine ; le citrate d'acétyltributyle ; le succinate de dibutyle ; l'huile de ricin hydrogénée ; les acides gras ; les triglycérides et glycérides substitués ; et des mélanges de ceux-ci.

8. Particule selon l'une quelconque des revendications précédentes, dans laquelle la première couche possède une valeur d'allongement à la rupture d'au moins environ 70%.

9. Particule selon l'une quelconque des revendications précédentes, dans laquelle la deuxième couche est constituée de copolymères d'esters d'acide acrylique.

10. Particule selon l'une quelconque des revendications précédentes, dans laquelle la deuxième couche est constituée d'un copolymère filmogène comprenant le produit de réaction d'acide polyméthacrylique et d'au moins un élément du groupe constitué par l'ester d'acide acrylique et l'ester d'acide polyméthacrylique.

11. Particule selon l'une quelconque des revendications précédentes, dans laquelle la première couche comprend, sur la base du poids total de la première couche, au moins environ 50% d'un polymère gastrorésistant.

12. Comprimé constitué des particules selon l'une quelconque des revendications précédentes.

13. Comprimé croquable constitué des particules selon l'une quelconque des revendications précédentes.

14. Comprimé croquable selon la revendication 13, dans lequel le plastifiant est choisi dans le groupe constitué par le polyéthylène glycol ; le propylène glycol ; le glycérol ; le sorbitol ; le citrate de triéthyle ; le citrate de tributyle ; le sébacate de dibutyle ; les huiles végétales ; les agents tensioactifs ; le mono-acétate de glycérol ; le diacétate de glycérol ; le triacétate de glycérol ; les gommes naturelles ; la triacétine ; le citrate d'acétyltributyle ; l'oxalate de diéthyle ; le malate de diéthyle ; le fumarate de diéthyle ; le malonate de diéthyle ; le phtalate de dioctyle ; le succinate de dibutyle ; le tributyrate de glycérol ; l'huile de ricin hydrogénée ; les acides gras ; les triglycérides et glycérides substitués ; et des mélanges de ceux-ci.

15. Comprimé croquable selon la revendication 13, dans lequel la première couche comprend, sur la base du poids total de la première couche, au moins environ 20% d'un polymère gastrorésistant.

16. Forme de dosage fondante constituée des particules selon l'une des revendications 1-11.

17. Comprimé croquable constitué d'au moins une première particule selon l'une quelconque des revendications 1-11 et d'au moins une deuxième particule ;
ladite deuxième particule comprenant un coeur de deuxième particule contenant un deuxième ingrédient actif.
